# EUROPÄISCHE PATENTANMELDUNG

(11) **EP 1 273 279 A2**
(43) Veröffentlichungstag der Anmeldung: **08.01.2003**
(21) Anmeldenummer: 02010031.9
(22) Anmeldetag: 06.05.2002
(51) Int. Cl.: A61F 9/01

(54) **Messeinrichtung für Strahlparameter der Bearbeitungsstrahlung einer Vorrichtung zur fotorefraktiven Korrektur von Fehlsichtigkeiten sowie Vorrichtung zur fotorefraktiven Korrektur von Fehlsichtigkeiten**

(30) Priorität: 15.06.2001 DE 10128650
(71) Anmelder: Jurca Optoelektronik GmbH & Co. KG, 63110 Rodgau 1 (DE)
(72) Erfinder: Dietz, Christoph, 63170 Obertshausen (DE); Palzer, Gabriel, 63263 Neu-Isenburg (DE); Mokler, Hans-Dieter, 63571 Gelnhausen (DE); Marschall, Michael, 63069 Offenbach (DE); Banszerus, Manfred, 63165 Mühlheim (DE)
(74) Vertreter: Ostertag, Ulrich, Dr.

(57) **Zusammenfassung**

Zur Vermessung der Strahlparameter der Behandlungsstrahlung (2) einer Vorrichtung (1) zur fotorefraktiven Korrektur von Fehlsichtigkeiten ist eine Meßeinrichtung vorgesehen. Diese umfaßt ein in dem Behandlungsbereich anstelle der zu behandelnden Augenhornhaut angeordneten und mit der Behandlungsstrahlung (2) beaufschlagbaren und bei Beaufschlagung mit Behandlungsstrahlung (2) Meßstrahlung (13) emittierendes Medium (11). Ferner weist die Meßeinrichtung eine Abbildungsoptik (22) auf, die den Beaufschlagungsbereich auf einen positionsempfindlichen Strahlungssensor (23) abbildet. Dieser erfaßt die Intensitätsverteilung der Meßstrahlung (13) über deren Querschnitt. Die Vorrichtung (1) weist zusätzlich eine Nachführvorrichtung (5 bis 9) zum kontinuierlichen Nachführen des Behandlungsstrahls (2) in der Behandlungsebene (10) auf, die mit einer eine Recheneinheit aufweisenden Steuereinrichtung (18) zusammenarbeitet. Der positionsempfindliche Strahlungssensor (23) steht mit der Steuereinrichtung (18) zum Vergleich einer sich aus den Daten der Nachführvorrichtung (5 bis 9) ergebenden Behandlungsposition mit einer sich aus den Daten des positionsempfindlichen Strahlungssensors (23) ergebenden Bearbeitungsposition in der Recheneinheit in Signalverbindung. Eine derartige Meßeinrichtung in der Vorrichtung (1) erlaubt eine präzise Vermessung der Strahlparameter der Behandlungsstrahlung (2).

## Beschreibung

Die Erfindung betrifft eine Meßeinrichtung für Strahlparameter der Behandlungsstrahlung einer Vorrichtung zur fotorefraktiven Korrektur von Fehlsichtigkeiten mit einem in dem Behandlungsbereich anstelle der zu behandelnden Augenhornhaut angeordneten und mit der Behandlungsstrahlung beaufschlagbaren und bei Beaufschlagung mit Behandlungsstrahlung Meßstrahlung emittierenden Medium.

Ferner betrifft die Erfindung eine Vorrichtung zur fotorefraktiven Korrektur von Fehlsichtigkeiten mit einer Nachführvorrichtung zum kontinuierlichen Nachführen des Behandlungsstrahls in der Behandlungsebene, wobei die Nachführvorrichtung mit einer eine Recheneinheit aufweisenden Steuereinrichtung zusammenarbeitet.

Eine Meßeinrichtung für eine derartige Vorrichtung, die in der DE 196 18 883 A1 beschrieben ist, ist vom Markt her bekannt.

Bei derartigen bekannten Vorrichtungen wird eine Gelatine-Folie vorgegebener Dicke so lange mit gepulster Behandlungs-bzw. Bearbeitungsstrahlung beaufschlagt, bis ein Durchbruch der Gelatine-Folie erfolgt. Dieser Durchbruch wird mit einem Sensor detektiert, in dem das Fluoreszenzlicht einer in Strahlrichtung nach der Gelatine-Folie angeordneten Detektorscheibe gemessen wird. Die Dicke der Gelatine-Folie dividiert durch die bis zum Durchbruch benötigte Anzahl der Behandlungsstrahlimpulse ist ein Maß für die Ablation pro Puls, die wiederum von der Impulsenergie abhängt.

Eine solche Impulsenergiemessung des Behandlungsstrahls, die zur Kalibrierung der Vorrichtung notwendig ist, erfordert, daß die Dicke der Gelatine-Folie genau bekannt ist und zudem, daß diese über ihre Dicke eine homogene Zusammensetzung aufweist. Die bekannten Gelatine-Folien weisen jedoch eine gewisse Streuung in ihrer Dicke auf, worunter die Genauigkeit der Energiemessung und damit der Kalibrierung der Gesamtvorrichtung leiden. Zudem sind Gelatine-Folien hygroskopisch, so daß auch die Bedingung der homogenen Zusammensetzung in der Regel nicht erfüllt ist, was die Messung zusätzlich verfälscht. Außerdem absorbiert der aufsteigende Dampf der ablatierten Gelatine-Folie den Behandlungsstrahl teilweise, was eine weitere Verringerung der Meßgenauigkeit mit sich bringt.

Es ist daher die Aufgabe der vorliegenden Erfindung, eine Meßeinrichtung der eingangs genannten Art derart weiterzubilden, daß eine präzise Messung der Strahlparameter der Behandlungsstrahlung ermöglicht ist, die eine genaue und reproduzierbare Kalibrierung der Gesamtvorrichtung zuläßt.

Diese Aufgabe wird erfindungsgemäß gelöst durch eine Abbildungsoptik, die den Beaufschlagungsbereich auf einen positionsempfindlichen Strahlungssensor abbildet, welcher die Intensitätsverteilung der Meßstrahlung über deren Querschnitt erfaßt.

Als Meßstrahlung wird nachfolgend allgemein Strahlung bezeichnet, die einen Rückschluß auf Strahlparameter der Behandlungsstrahlung zuläßt. Die Meßstrahlung kann dabei die gleiche Wellenlänge wie die Behandlungsstrahlung aufweisen, insbesondere von der Behandlungsstrahlung abgezweigt sein, oder dieser gegenüber wellenlängenverschoben sein.

Die Erfindung fußt auf der Idee, für die Strahlparametermessung eine Sensortechnik, nämlich einen positionsempfindlichen Strahlungssensor, einzusetzen, die im Rahmen des Nachführens der Behandlungsstrahlung relativ zum zu behandelnden Beaufschlagungsbereich bei Vorrichtungen zur fotorefraktiven Korrektur schon eingesetzt ist. Die bisher verwendeten positionsempfindlichen Strahlungssensoren waren aber so ausgewählt und angeordnet, daß sie für Meßstrahlung, die Strahlparameterinformation enthält, nicht sensitiv waren. Vielmehr wurde dort eine Strahlung erfaßt, die von der Behandlungsstrahlung unabhängig war und daher keine Rückschlüsse auf Strahlparameter der Behandlungsstrahlung zuließ. Erfindungsgemäß wird ein meßstrahlungssensitiver positionsempfindlicher Strahlungssensor eingesetzt mit dem die Gesamtenergie des Behandlungsstrahls sowie sein Intensitätsprofil gemessen werden kann, wobei wahlweise der schon vorhandene positionsempfindliche Strahlungssensor so abgewandelt wird, daß er für Meßstrahlung sensitiv ist, oder ein unabhängiger weiterer positionsempfindlicher Strahlungssensor eingesetzt wird.

Mit einem positionsempfindlichen Strahlungssensor läßt sich, eine entsprechende Kalibrierung des Sensors vorausgesetzt, die Gesamtenergie der Behandlungsstrahlung messen. Zudem lassen sich, abhängig von der Wirkung der Abbildungsoptik, noch weitere Strahlungsparameter, nämlich z.B. die Position des Behandlungsstrahls in der Behandlungsebene und die Kontur des Behandlungsstrahls in der Behandlungsebene ermitteln. Die Strahlparametermessung ist nicht auf eine gepulste Behandlungsstrahlung beschränkt, sondern kann auch bei kontinuierlicher Behandlungsstrahlung durchgeführt werden.

Der positionsempfindliche Strahlungssensor kann eine Matrix aus strahlungsempfindlichen Sensorelementen, insbesondere ein CCD-Array umfassen. Ein solcher Strahlungssensor weist eine hohe Positionsauflösung auf. CCD-Arrays sind zudem sehr empfindlich.

Bei einer bevorzugten Ausführungsform ist mindestens ein weiterer, nicht positionsempfindlicher Strahlungssensor vorgesehen, der dem Medium benachbart angeordnet ist und die Gesamtenergie der Meßstrahlung erfaßt. Mit einem solchen zusätzlichen Strahlungssensor läßt sich ein Meßwert für die Gesamtenergie der Bearbeitungsstrahlung generieren, welcher von der Führung der Behandlungsstrahlung durch die Abbildungsoptik unabhängig ist. Dies erhöht die Sicherheit bei der Bestimmung der Strahlungsenergie.

Das Medium kann ein scheibenförmiger Körper mit einer Eintrittsfläche für Bearbeitungsstrahlung und einer Umfangsfläche sein, wobei der mindestens eine Strahlungssensor Meßstrahlung erfaßt, die durch die Umfangsfläche aus dem Körper austritt. Bei einer derartigen Anordnung des mindestens einen nicht positionsempfindlichen Strahlungssensors ist sichergestellt, daß sich die Erfassungskegel des positionsempfindlichen Strahlungssensors und des mindestens einen nicht positionsempfindlichen Strahlungssensors nicht obstruieren. Ein Beispiel für ein scheibenförmiges Medium, welches bei Beaufschlagung mit Behandlungsstrahlung Meßstrahlung emittiert, ist eine unter UV-Licht-Beaufschlagung fluoreszierende Saphirglasscheibe. Insgesamt ist auch bei Einsatz mehrerer nicht positionsempfindlicher Strahlungssensoren eine kompakte Anordnung des Körpers und des zugeordneten nicht positionsempfindlichen Strahlungssensoren möglich.

Der scheibenförmige Körper kann als dotierte Saphirscheibe ausgebildet sein. Dieses Material eignet sich zur zuverlässigen und genauen Kalibrierung des Bearbeitungsstrahls.

Vorzugsweise ist eine Mehrzahl von um die Umfangsfläche des Körpers herum angeordneten nicht positionsempfindlichen Strahlungssensoren vorgesehen. Hierdurch kann insbesondere der gesamte Umfang des scheibenförmigen Körpers zur Strahlungsparametermessung abgedeckt werden. Die Erfassung der Meßstrahlung mit einer derartigen Mehrzahl von Strahlungssensoren führt zur Möglichkeit der Mittelung der Meßwerte. Fehlerquellen wie eine sich ändernde Sensitivität einzelner nicht positionsempfindlicher Strahlungssensoren oder eine sich ändernde Transmission in Abschnitten der Umfangsfläche des Körpers oder weitere das Meßergebnis einzelner Strahlungssensoren beeinflussender Faktoren lassen sich auf diese Weise in ihrem Einfluß verringern.

Eine weitere Aufgabe der Erfindung ist es, eine Vorrichtung zur fotorefraktiven Korrektur von Fehlsichtigkeiten der eingangs genannten Art derart weiterzubilden, daß eine präzise Messung der Strahlparameter der Behandlungsstrahlung ermöglicht ist, die eine genaue und reproduzierbare Kalibrierung der Gesamtvorrichtung zuläßt.

Diese Aufgabe wird gelöst durch eine Vorrichtung mit einer erfindungsgemäßen Meßeinrichtung. Die Vorrichtung hat die im Zusammenhang mit der Meßeinrichtung beschriebenen Vorteile.

Bei der Vorrichtung kann der positionsempfindliche Strahlungssensor mit der Steuereinrichtung zum Vergleich einer sich aus den Daten der Nachführvorrichtung ergebenden Bearbeitungsposition mit einer sich aus den Daten des positionsempfindlichen Strahlungssensors ergebenden Behandlungsposition in der Recheneinheit in Signalverbindung stehen.

Bei einer derart weitergebildeten Vorrichtung zur fotorefraktiven Korrektur von Fehlsichtigkeiten wird die schon vorhandene Nachführvorrichtung in die Strahlparametermessung integriert. Läßt sich beispielsweise eine bestimmte Position in der Behandlungsebene nur durch ein extremes Verstellen der Nachführvorrichtung herstellen, so ist dies über einen Vergleich der mit der Meßeinrichtung gemessenen Position der Behandlungsstrahlung in der Behandlungsebene mit der zugeordneten Einstellung der Nachführvorrichtung ermittelbar. Auf diese Weise können ein zeitlicher Drift der Behandlungsstrahlung, bei dem zur Beibehaltung einer bestimmten Position der Behandlungsstrahlung in der Behandlungsebene eine immer stärkere Verstellung der Nachführvorrichtung erforderlich ist, oder eine Dejustage der Strahlführungskomponenten für die Behandlungsstrahlung, bei der die Position der Behandlungsstrahlung in der Behandlungsebene nicht oder nur mit einer hierfür nicht vorgesehenen Einstellung der Nachführvorrichtung erreicht wird, detektiert und entsprechende Gegenmaßnahmen ergriffen werden.

Bevorzugt steht die Steuereinrichtung mit einer Strahlführungsoptik für die Behandlungsstrahlung zur Beeinflussung der Strahlparameter in Signalverbindung. Über die Strahlformungsoptik lassen sich, entsprechende Komponenten in der Strahlformungsoptik vorausgesetzt, praktisch alle Strahlparameter beeinflussen. Hierfür können in Richtung der optischen Achse der Strahlformungsoptik und lateral hierzu justierbare Linsen, einstellbare Filter oder auch eine einstellbare Lochmaskenvorrichtung vorgesehen sein.

Alternativ oder zusätzlich kann die Steuereinrichtung mit der Quelle für die Behandlungsstrahlung zur Beeinflussung der Strahlparameter in Signalverbindung stehen. Je nach Art der eingesetzten Strahlungsquelle läßt sich durch entsprechendes Ansteuern von dieser die Energie oder auch die Strahlform der Behandlungsstrahlung flexibel beeinflussen.

Ein Ausführungsbeispiel der Erfindung wird nachfolgend anhand der Zeichnung näher erläutert; es zeigen:
- Figur 1: eine schematische Darstellung einer Vorrichtung zur fotorefraktiven Korrektur von Fehlsichtigkeiten;
- Figur 2: eine Aufsicht auf Komponenten einer Meßrichtung für Strahlparameter einer Behandlungsstrahlung der Vorrichtung gemäß Linie II-II von Figur 1; und
- Figur 3: eine vergrößerte Darstellung einer Fluoreszenzscheibe, die zu den in Figur 2 dargestellten Komponenten gehört.

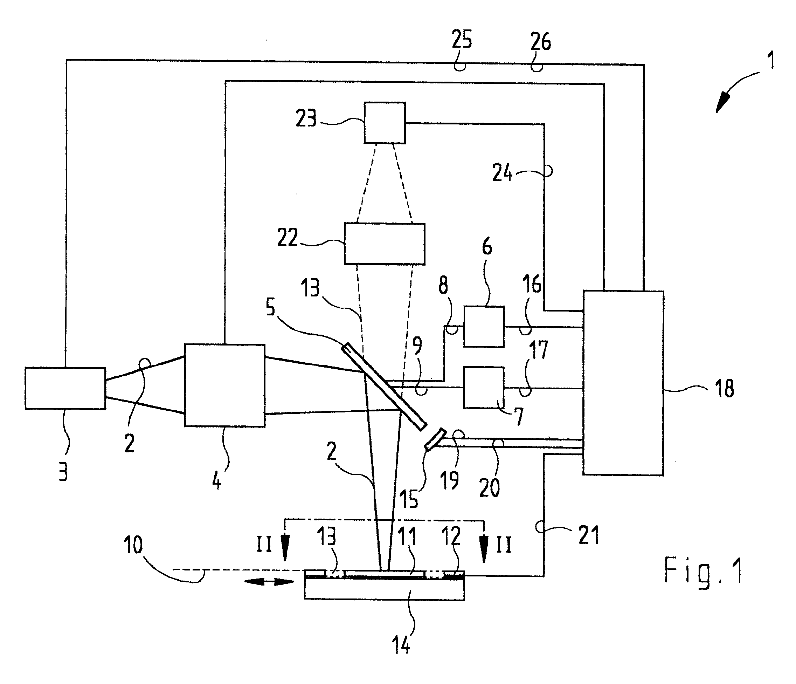

Die Vorrichtung zur fotorefraktiven Korrektur von Fehlsichtigkeiten, die in Figur 1 schematisch dargestellt und insgesamt mit dem Bezugszeichen 1 versehen ist, ist, was die Erzeugung und die Führung eines Behandlungsstrahls 2 angeht, aus der DE 196 18 883 A1 bekannt, auf die in diesem Zusammenhang Bezug genommen wird.

Ein UV-Laser erzeugt den gepulsten Behandlungsstrahl 2, der eine Wellenlänge von 193 nm aufweist. Eine Strahlformoptik 4, die ein Linsensystem, eine Komponente zur Homogenisierung des Behandlungsstrahls 2 über dessen Querschnitt und eine Lochmaskenvorrichtung umfaßt, formt den Behandlungsstrahl 2, damit dieser eine zur fotorefraktiven Korrektur geeignete Bündeldivergenz bzw. -konvergenz und eine geeignete Kontur aufweist.

Nach dem Durchtreten der Strahlformoptik 4 wird der Behandlungsstrahl 2 von einem kardanisch gelagerten Umlenkspiegel 5 um ca. 90° umgelenkt. Der Umlenkspiegel 5 weist eine dichroitische Beschichtung derart auf, daß die Wellenlänge des Behandlungsstrahls 2 vollständig reflektiert wird. Über zwei Aktuatoren 6 bzw. 7, die mit dem Umlenkspiegel 5 über schematisch angedeutete Gestänge 8 bzw. 9 verbunden sind, ist der Umlenkspiegel 5 um zwei zueinander senkrechte und in der Reflexionsebene des Umlenkspiegels 5 liegende Kippachsen verkippbar.

Über Signalleitungen 16 bzw. 17 sind die Aktuatoren 6 bzw. 7 mit einer zentralen Steuereinrichtung 18 der Vorrichtung 1 verbunden. Mit einem Stellungssensor 15 kann die momentane Verkippung des Umlenkspiegels 5 um die beiden Kippachsen gemessen werden. Die Werte der momentanen absoluten Verkippungen um die beiden Kippachsen übermittelt der Stellungssensor 15 der Steuereinrichtung 18 über Signalleitungen 19 bzw. 20. Durch Betätigen der Aktuatoren 6, 7 läßt sich somit die Richtung des Behandlungsstrahls 2 nach dem Umlenkspiegel 5 so beeinflussen, daß der Behandlungsstrahl 2 gemäß einem Vorgabewert nachgeführt werden kann, wie dies in der DE 196 18 883 A1 beschrieben ist.

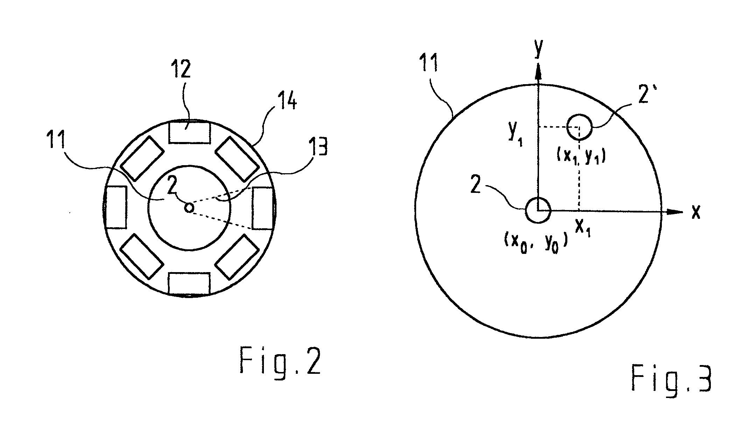

Bei der fotorefraktiven Korrekturbehandlung trifft der Behandlungsstrahl 2 in einer Behandlungsebene 10 auf die zu behandelnde Hornhaut (in Figur 1 nicht dargestellt). In der Figur 1 ist die Vorrichtung 1 in einem Kalibriermodus dargestellt, in dem am Ort des zu behandelnden Auges eine Fluoreszenzscheibe 11 aus dotiertem Saphirglas angeordnet ist, die Teil einer Meßeinrichtung für Strahlparameter des Behandlungsstrahls 2 ist. Um die optisch polierte Umfangsfläche der runden Fluoreszenzscheibe 11 herum sind, wie insbesondere aus Figur 2 ersichtlich, insgesamt acht Fotodioden 12 angeordnet. Die Fotodioden 12 sind empfindlich für Fluoreszenzstrahlung 13, die in den Figuren 1 und 2 gestrichelt dargestellt ist, wobei in Figur 2 beispielhaft nur der Fluoreszenzstrahlungserfassungskegel einer der acht Fotodioden 12 eingezeichnet ist.

Die Fotodioden 12 sind mit der Steuereinrichtung 18 über Signalleitungen verbunden, von denen in Figur 1 stellvertretend die Signalleitung 21 gezeigt ist. Die Fluoreszenzscheibe 11 und die Fotodioden 12 sind gemeinsam auf einem Träger 14 angeordnet, der parallel zur Bearbeitungsebene 10 verschiebbar ist, so daß die Fluoreszenzscheibe 11 mit den Fotodioden 12 zur noch zu beschreibenden Vermessung der Strahlparameter in den Strahlengang des Behandlungsstrahls 2 hinein- und herausschiebbar ist.

Die dichroitische Beschichtung des Umlenkspiegels 5 ist für die Fluoreszenzstrahlung 13 durchlässig. Eine bezogen auf die Behandlungsebene 10 hinter dem Umlenkspiegel 5 liegende Abbildungsoptik 22 bildet die Behandlungsebene 10 auf eine Erfassungsebene eines CCD-Arrays 23 ab. Dieses steht über eine Signalleitung 24 mit der Steuereinrichtung 18 in Verbindung.

Das CCD-Array 23 dient neben den hier erläuterten Meßzwecken während der fotorefraktiven Augenbehandlung der Verfolgung der Augenposition, wie dies in der DE 196 18 883 A1 beschrieben ist.

Über eine Signalleitung 25 steht die zentrale Steuereinrichtung 18 mit dem UV-Laser 3 und über eine Signalleitung 26 mit der Strahlformoptik 4 in Verbindung.

Das gesamte Strahlführungssystem der Korrekturvorrichtung 1 kann mit hochreinem Stickstoff gespült werden, um die Absorption der Behandlungsstrahlung 2 zu verringern.

Bei einer alternativen, nicht in der Zeichnung dargestellten Ausführung der Korrekturvorrichtung 1 ist der dichroitische Umlenkspiegel 5 nicht beweglich. Vor diesem sind bei dieser Ausführungsform im Strahlengang der Behandlungsstrahlung 2 zwei separate Scanner-Einrichtungen angeordnet, die analog zum Umlenkspiegel 5 eine Umlenkung des Behandlungsstrahls 2 in zwei zueinander senkrechten Richtungen bewirken. Die Scanner-Einrichtungen können als Galvanometer-Scanner ausgeführt sein.

Die Strahlparameter des Behandlungsstrahls 2 werden wie folgt vermessen:

Die in den Strahlengang des Behandlungsstrahls 2 eingeschobene Fluoreszenzscheibe 11 fluoresziert von dort, wo sie vom Behandlungsstrahl 2 durchtreten wird, wellenlängenverschobene Fluoreszenzstrahlung 13 in alle Raumrichtungen. Es entsteht in der Fluoreszenzscheibe daher ein Fluoreszenzkanal, dessen Form und Fluoreszenzintensitätsverteilung die Strahlparameter des Behandlungsstrahls 2 charakterisiert.

Die durch die Umfangsfläche der Fluoreszenzscheibe 11 austretende Fluoreszenzstrahlung 13 wird von den Fotodioden 12 erfaßt. Der über alle Fotodioden 12 gemittelte Strahlungswert für die Fluoreszenzstrahlung 13 ist ein präzises Maß für die Gesamtenergie des Behandlungsstrahls 2.

Die Fluoreszenzstrahlung 13, die in Richtung des Umlenkspiegels 5 ausgesandt wird, durchtritt diesen. Die Abbildungsoptik 22 bildet die Bearbeitungsebene 10 auf das CCD-Array 23 ab, so daß über den Querschnitt des Fluoreszenzkanals die Kontur und die Intensitätsverteilung des Behandlungsstrahls 2 in der Behandlungsebene 10 vom CCD-Array 23 erfaßt werden kann. Die Fotodioden 12 und das CCD-Array 23 bilden daher redundante Einrichtungen zur Messung der Gesamtenergie des Behandlungsstrahls. Hierdurch wird, wie für medizinische Anwendungen gefordert, die Betriebssicherheit der Korrekturvorrichtung 1 erhöht.

Die Abbildungsoptik 22 ist so ausgeführt, daß das CCD-Array 23 den Fluoreszenzkanal, der den Behandlungsstrahl 2 charakterisiert, auch innerhalb der Fluoreszenzscheibe 11 verfolgen kann, so daß auch die Konvergenz bzw. Divergenz des Behandlungsstrahls 2 im Bereich der Behandlungsebene 10 vermessen werden kann. Zudem ist über den Auftreffpunkt der abgebildeten Fluoreszenzstrahlung 13 auf dem CCD-Array 23 eine Messung der Lage des Behandlungsstrahls 2 in der Behandlungsebene 10 möglich.

Die Meßdaten der Fotodioden 12 einerseits und des CCD-Arrays 23 andererseits werden über die Signalleitungen 21 bzw. 24 der zentralen Steuereinrichtung 18 zugeführt.

Die Strahlparameter-Meßdaten werden in einer Recheneinheit der Steuereinrichtung 18 verarbeitet. Die Steuereinrichtung 18 erzeugt in Abhängigkeit von den Strahlparameter-Meßdaten Steuerwerte, die über die Signalleitungen 25 bzw. 26 an den UV-Laser 3 bzw. die Strahlformoptik 4 übermittelt werden. Auf diese Weise kann die Steuereinrichtung 18 über die Signalleitung 25 die vom UV-Laser 3 emittierte Energie des Behandlungsstrahls 2 sowie die Richtung des emittierten Behandlungsstrahls 2 beeinflussen. Über die Ansteuerung der Strahlformoptik 4 läßt sich die Kontur des Behandlungsstrahls 2 sowie dessen Konvergenz bzw. Divergenz beeinflussen. Die Position des Behandlungsstrahls 2 in der Bildebene 10 läßt sich über den Umlenkspiegel 5 beeinflussen. Hierzu steuert die Steuereinrichtung 18 über die Signalleitungen 16 bzw. 17 die Aktuatoren 6 bzw. 7 entsprechend an.

In einem Speicher der Steuereinrichtung 18 sind Grenzwerte für die Strahlparameter Gesamtenergie, Intensitätsprofil und Position abgelegt. Verlassen die gemessenen Strahlparameter einen Toleranzbereich, der durch diese Grenzwerte vorgegeben ist, so wird der UV-Laser abgeschaltet.

Die Positionsmessung des Orts, an dem der Behandlungsstrahl 2 die Behandlungsebene 10 durchtritt, verdeutlicht Figur 3. Dort ist die Fluoreszenzscheibe 11 in Aufsicht vergrößert dargestellt. Die Sollposition des Behandlungsstrahls 2 ist im Zentrum eines die Bearbeitungsebene 10 aufspannenden kartesischen Koordinatensystems dargestellt; der Sollposition ist also das Wertepaar x₀, y₀ zugeordnet. Im ersten Quadranten des Koordinatensystems ist beispielhaft ein gegenüber der Sollposition verlagerter Behandlungsstrahl 2' mit einem Positionswertepaar x₁, y₁ gezeigt.

In einem Speicher der Steuereinrichtung 18 sind Grenzwerte für die Strahlparameter abgelegt. Verlassen sie gemessenen Strahlparameter einen Toleranzbereich, der durch diese Grenzwerte vorgegeben ist, so wird der UV-Laser abgeschaltet.

Durch Speicherung der Positionsdaten (Wertepaar x, y) des Behandlungsstrahls 2 in der Behandlungsebene 10 und der gleichzeitig gemessenen Positionswerte des Umlenkspiegels 5 über den Stellungssensor 15 ist die Vermessung von Drifts oder einer optischen Dejustage des Behandlungsstrahls 2 möglich, die bei Überschreiten einer bestimmten Größe nicht mehr durch Nachstellen des Umlenkspiegels 5 ausgeglichen werden kann. Hierzu wird von Zeit zu Zeit verglichen, ob einem gegebenen Wertepaar x, y des Behandlungsstrahls 2 in der Behandlungsebene 10 noch die entsprechenden Positionsdaten des Stellungssensor 15 zugeordnet sind.

Anstelle der Fotodioden 12 und des CCD-Arrays 23 sind auch andere Sensortypen zur Bestimmung der Gesamtenergie und/oder weiterer Strahlparameter des Behandlungsstrahls 2 einsetzbar, z.B. CMOS-Sensoren.

## Patentansprüche

1. Meßeinrichtung für Strahlparameter der Behandlungsstrahlung einer Vorrichtung zur fotorefraktiven Korrektur von Fehlsichtigkeiten mit einem in dem Behandlungsbereich anstelle der zu behandelnden Augenhornhaut angeordneten und mit der Behandlungsstrahlung beaufschlagbaren und bei Beaufschlagung mit Behandlungsstrahlung Meßstrahlung emittierenden Medium,
**gekennzeichnet durch**
eine Abbildungsoptik (22), die den Beaufschlagungsbereich auf einen positionsempfindlichen Strahlungssensor (23) abbildet, welcher die Intensitätsverteilung der Meßstrahlung (13) über deren Querschnitt erfaßt.

2. Meßeinrichtung nach Anspruch 1, **dadurch gekennzeichnet,**
**daß** der positionsempfindliche Strahlungssensor eine Matrix aus strahlungsempfindlichen Sensorelementen, insbesondere ein CCD-Array (23), umfaßt.

3. Meßeinrichtung nach Anspruch 1 oder 2, **gekennzeichnet**
**durch** mindestens einen weiteren, nicht positionsempfindlichen Strahlungssensor (12), der dem Medium (11) benachbart angeordnet ist und die Gesamtenergie der Meßstrahlung (13) erfaßt.

4. Meßeinrichtung nach Anspruch 3, **dadurch gekennzeichnet,**
**daß** das Medium ein scheibenförmiger Körper (11) mit einer Eintrittsfläche für Behandlungsstrahlung (2) und einer Umfangsfläche ist, wobei der mindestens eine Strahlungssensor (12) Meßstrahlung (13) erfaßt, die durch die Umfangsfläche aus dem Körper (11) austritt.

5. Meßeinrichtung nach Anspruch 4, **dadurch gekennzeichnet,**
**daß** der scheibenförmige Körper als dotierte Saphirscheibe ausgebildet ist.

6. Meßeinrichtung nach Anspruch 4 oder 5, **gekennzeichnet**
**durch** eine Mehrzahl von um die Umfangsfläche des Körpers (11) herum angeordneten nicht positionsempfindlichen Strahlungssensoren (12).

7. Vorrichtung zur fotorefraktiven Korrektur von Fehlsichtigkeiten mit einer Nachführvorrichtung zum kontinuierlichen Nachführen des Behandlungsstrahls in der Behandlungsebene, die mit einer eine Recheneinheit aufweisenden Steuereinrichtung zusammenarbeitet,
**dadurch gekennzeichnet, daß**
sie eine Meßeinrichtung nach einem der vorhergehenden Ansprüche enthält.

8. Vorrichtung nach Anspruch 7, **dadurch gekennzeichnet,**
**daß** der positionsempfindliche Strahlungssensor (23) mit der Steuereinrichtung (18) zum Vergleich einer sich aus den Daten der Nachführvorrichtung (5 bis 9) ergebenden Behandlungsposition mit einer sich aus den Daten des positionsempfindlichen Strahlungssensors (23) ergebenden Behandlungsposition in der Recheneinheit in Signalverbindung (24) steht.

9. Vorrichtung nach Anspruch 7 oder 8, **dadurch gekennzeichnet, daß** die Steuereinrichtung (18) mit einer Strahlformungsoptik (4) für die Behandlungsstrahlung (2) zur Beeinflussung der Strahlparameter in Signalverbindung (26) steht.

10. Vorrichtung nach einem der Ansprüche 7 bis 9, **dadurch gekennzeichnet, daß** die Steuereinrichtung (18) mit der Quelle (3) für die Behandlungsstrahlung (2) zur Beeinflussung der Strahlparameter in Signalverbindung (25) steht.

11. Vorrichtung nach Anspruch 10, **dadurch gekennzeichnet,**
**daß** die Steuereinrichtung (18) so ausgeführt ist, daß die Zufuhr von Behandlungsstrahlung (2) zum Behandlungsbereich unterbricht, wenn gemessene Strahlparameter der Behandlungsstrahlung (2) vorbestimmte Wertebereiche verlassen.
